(19) Europäisches Patentamt
European Patent Office
Office européen des brevets

(11) Veröffentlichungsnummer: **0 131 875**
**B1**

(12) **EUROPÄISCHE PATENTSCHRIFT**

(45) Veröffentlichungstag der Patentschrift: **16.12.87**

(21) Anmeldenummer: **84107951.0**

(22) Anmeldetag: **06.07.84**

**E R R A T U M**

| DIE TEXTSTELLE : TEXT PUBLISHED : LE PASSAGE SUIVANT : | (SEITE, SPALTE, ZEILE) (PAGE, COLUMN, LINE) (PAGE, COLONNE, LIGNE) | | | LAUTET BERICHTIGT: SHOULD READ : DEVRAIT ETRE LU : |
|---|---|---|---|---|
| Verfabren zu ihrer Herstellung | 2 | | 3 | Verfahren zu ihrer Herstellung |
| $R^1 C_1-C_4$-Akyl | | | 23 | $R^1 C_1-C_4$-Alkyl |
| Die Ümsetzung | 3 | | 54 | Die Umsetzung |
| Formel $R_2O-NH_2$ | 4 | | 2 | Formel $R^2O-NH_2$ |
| $C_2CH = CH_2$ | 7 | Ex.42 | 8 | $CH_2CH = CH_2$ |
| $CH_2 = CH_2$ | | Ex.47 | 13 | $CH_2CH = CH_2$ |
| 6-OC6$H_4$Cl | | Ex.72 | 38 | 6-$OC_6H_4Cl$ |
| 1166 | 8 | | 16 | 166 |
| 4-$OCH_2H_5$ | | Ex.167 | 17 | 4-$OC_2H_5$ |
| $C_2M_5$ | 10 | Ex.208 | 21 | $C_2H_5$ |
| bei-Spielsweise | | | 59 | beispielsweise |
| stsrk polare Lösungsmittel | 11 | | 5 | stark polare Lösungsmittel |
| die Wirkstofie | 12 | | 7 | die Wirkstoffe |
| als Substituent tragt | 14 | | 57 | als Substituent trägt |
| $N_D^{26}$ | 10 | | 35 | $n_D^{26}$ |

Tag der Entscheidung über die Berichtigung / Date of decision on rectification / Date de décision portant sur modification: 01.02.88

Ausgabe- und Veröffentlichungstag / Issue and publication date / Date d'edition et de publication: 06.04.88

Patbl.Nr / EPB no / Bull. no: 88/14

(19) Europäisches Patentamt
European Patent Office
Office européen des brevets

(11) Veröffentlichungsnummer: **0 131 875**
**B1**

(12) **EUROPÄISCHE PATENTSCHRIFT**

(45) Veröffentlichungstag der Patentschrift: **16.12.87**

(21) Anmeldenummer: **84107951.0**

(22) Anmeldetag: **06.07.84**

(51) Int. Cl.⁴: **C 07 D 309/06**, C 07 D 309/10, C 07 D 309/08, C 07 D 335/02, C 07 D 319/12, C 07 D 307/20, A 01 N 43/16, A 01 N 43/18, A 01 N 43/08, A 01 N 43/24

(54) **Cyclohexan-1,3-dionderivate, Verfahren zu ihrer Herstellung und ihre Verwendung zur Bekämpfung unerwünschten Pflanzenwuches.**

(30) Priorität: **08.07.83 DE 3324707**

(43) Veröffentlichungstag der Anmeldung: **23.01.85 Patentblatt 85/4**

(45) Bekanntmachung des Hinweises auf die Patenterteilung: **16.12.87 Patentblatt 87/51**

(84) Benannte Vertragsstaaten: **AT BE CH DE FR GB IT LI NL**

(56) Entgegenhaltungen:
**EP-A-0 070 370**
**EP-A-0 071 707**
**DE-A-2 439 104**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Becker, Rainer, Dr., Im Haseneck 22, D-6702 Duerkheim (DE)**
Erfinder: **Jahn, Dieter, Dr., Burgunderweg 8, D-6803 Edingen- Neckarhausen (DE)**
Erfinder: **Keil, Michael, Dr., Fontanestrasse 4, D-6713 Freinsheim (DE)**
Erfinder: **Himmele, Walter, Dr., Eichenweg 14, D-6909 Walldorf (DE)**
Erfinder: **Wuerzer, Bruno, Dr. Dipl.- Landwirt, Ruediger- Strasse 13, D-6701 Otterstadt (DE)**

EP 0 131 875 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft Cyclohexan-1,3-dionderivate, Verfabren zu ihrer Herstellung sowie Herbizide, die diese Verbindungen als Wirkstoffe enthalten.

Es ist bekannt, Cyclohexan-1,3-dionderivate zur Bekämpfung von unerwünschten Gräsern anzuwenden (DE-OS 31 21 355).

Es wurde nun gefunden, daß Cyclohexan-1,3-dionderivate der Formel

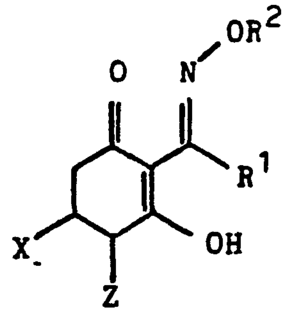

(I),

in der

$R^1$ $C_1$-$C_4$-Akyl,

$R^2$ $C_1$-$C_2$-Alkyl, gegebenenfalls halogensubstituiertes $C_3$-$C_5$-Alkenyl oder $C_3$-$C_5$-Alkinyl,

X einen gegebenenfalls ungesättigten fünf- oder sechsgliedrigen Heterocyclus, der mindestens einen $C_1$-$C_4$-Alkoxy-, $C_1$-$C_4$-Alkylthio-, $C_5$-$C_7$-Cycloalkoxy- oder Benzoxyrest oder einen gegebenenfalls substituierten Phenyl-, Phenoxy- oder Phenylthiorest als Substituent trägt und außerdem durch Alkylreste substituiert sein kann, und

Z Wasserstoff, Methoxycarbonyl, Ethoxycarbonyl, Methyl oder Cyano bedeuten,

oder Salze dieser Verbindungen eine vorzügliche Wirkung gegen Pflanzen aus der Familie der Gräser haben und dabei gut selektiv sind in breitblättrigen Kulturen sowie in solchen Kulturen, die zwar einkeimblättrig sind, aber nicht zu den Gräsern gehören. Darüber hinaus finden sich unter den Verbindungen der Formel I solche mit guter Wirkung gegen Gräser, die aber gleichzeitig auch noch eine Selektivität in grasartigen Kulturpflanzen, wie z. B. Getreidearten, haben.

Die Verbindungen der Formel I können in tautomeren Formen auftreten, die alle vom Patentanspruch umfaßt werden:

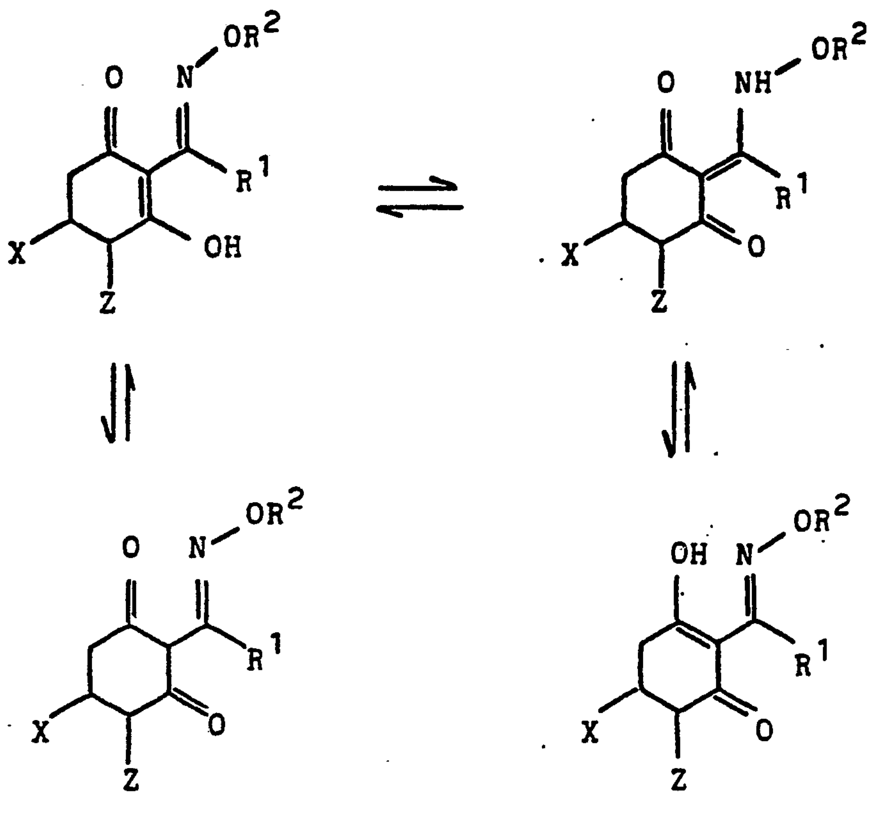

In der allgemeinen Formel I der Cyclohexan-1,3-dionderivate haben die Substituenten beispielsweise folgende Bedeutungen:

$R^1$ verzweigtes oder unverzweigtes Alkyl mit 1 bis 4 C-Atomen, wie Methyl, Ethyl, n-Propyl, iso-Propyl, n-Butyl, sec.-Butyl, iso-Butyl, tert.-Butyl,

$R^2$ verzweigtes oder unverzweigtes Alkyl mit 1 bis 4 C-Atomen, gegebenenfalls halogensubstituiertes Alkenyl mit 3 bis 5 C-Atomen oder Alkinyl mit 3 bis 5 C-Atomen, wie Methyl, Ethyl, n-Propyl, iso-Propyl, n-Butyl, sec.-Butyl, iso-Butyl, tert.-Butyl, Allyl, 3-Chlor-prop-2-enyl, 2-Chlor-prop-2-enyl, 1,2-Dichlor-prop-2-enyl, 1,1,2-Trichlor-prop-2-enyl, Propargyl;

X einen gegebenenfalls ungesättigten, vorzugweise maximal eine Doppelbindung enthaltenden, fünf- oder sechsgliedrigen Heterocyclus mit bis zu zwei Heteroatomen, ausgewählt aus der Gruppe, bestehend aus O,N und S, wie Tetrahydropyran-, Tetrahydrothiopyran-, Tetrahydrofuran- oder 1,4-Dioxanreste, beispielsweise Tetrahydropyran-2-yl, Tetrahydropyran-3-yl, Tetrahydrothiopyran-2-yl, Tetrahydrothiopyran-3-yl, Tetrahydrofuran-2-yl, Tetrahydrofuran-3-yl, 1,4-Dioxan-3-yl, jeweils substituiert durch $C_1$-$C_4$-Alkoxy, wie Methoxy, Ethoxy iso-Propoxy, n-Propoxy, n-Butoxy, sec.-Butoxy, tert.-Butoxy, durch $C_1$-$C_4$-Alkylthio, wie Methylthio, Ethylthio, iso-Propylthio, n-Propylthio, n-Butylthio, sec.-Butylthio, iso-Butylthio, tert.-Butylthio, durch $C_5$-$C_7$-Cycloalkoxy, wie Cyclopentoxy, Cyclohexoxy oder durch Benzoxy oder durch gegebenenfalls durch Halogen, $C_1$-$C_4$-Alkyl oder $C_1$-$C_4$-Alkoxy substituiertes Phenyl, Phenoxy oder Phenylthio, wie Phenyl, 4-Chlorphenyl, 4-Methylphenyl, 4-Fluorphenyl, 4-Methoxy-phenyl, Phenoxy, 4-Chlorphenoxy, 4-Fluorphenoxy, 4-Methoxyphenoxy, 4-Methylphenoxy, Phenylthio, 4-Chlorphenylthio, 4-Fluorphenylthio, 4-Methylphenylthio, 4-Methoxyphenylthio und gegebenenfalls außerdem substituiert durch bis zu drei Alkylresten, wie Methyl, Ethyl, n-Propyl, iso-Propyl, n-Butyl, iso-Butyl, sec.-Butyl, tert.-Butyl;

Z Wasserstoff, Methoxycarbonyl, Ethoxycarbonyl, Methyl oder Cyano.

Bevorzugte Verbindungen der Formel I sind solche, bei denen X einen gegebenenfalls ungesättigten fünf- oder sechsgliedrigen Heterocyclus, der mindestens einen $C_1$-$C_4$-Alkoxyrest als Substituenten trägt, bedeutet. Insbesondere bevorzugt sind Verbindungen der Formel I, bei denen X ein Tetrahydropyranylrest ist, und solche, bei denen Z Wasserstoff bedeutet.

Als Salze der Cyclohexan-1,3-dionderivate der Formel I kommen beispielsweise Alkalimetallsalze, wie Kalium- oder Natriumsalze, Erdalkalimetallsalze, wie Calcium-, Barium- oder Magnesiumsalze, weiterhin Mangan-, Kupfer-, Zink- oder Eisensalze sowie Ammoniumsalze, Phosphoniumsalze, Sulfonium- oder Sulfoxoniumsalze in Betracht, z. B. Tetraalkylammonium-, Trialkylphosphonium-, Trialkylsulfonium- oder Trialkylsulfoxoniumsalze, wie Tetramethylammonium-, Tetraethylammonium-, Trimethylphosphonium-, Trimethylsulfonium- oder Trimethylsulfoxoniumsalze.

Die Cyclohexan-1,3-dionderivate der Formel I können durch Umsetzung von Verbindungen der Formel

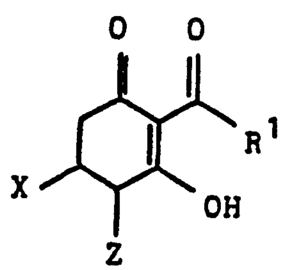 (II),

in der

$R^1$, X und Z die obengenannten Bedeutungen haben, mit Hydroxylaminderivaten der Formel $R^2O$-$NH_3Y$, in der $R^2$ die obengenannten Bedeutungen hat und Y ein beliebiges Anion bedeutet, erhalten werden.

Man führt die Reaktion zweckmäßigerweise in heterogener Phase in einem inerten Verdünnungsmittel bei einer Temperatur zwischen 0 und 80°C oder zwischen 0°C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Die Umsetzung verläuft besonders gut in einem pH-Bereich von 2 bis 9, insbesondere von 4,5 bis 5,5. Die Einstellung des pH-Bereiches erfolgt zweckmäßigerweise durch Zusatz von Acetaten, beispielsweise Alkalimetallacetaten, insbesondere von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^2O$-$NH_3Y$, zugesetzt.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, oder Ether, wie Dioxan, Tetrahydrofuran, geeignet. Auch Gemische dieser Lösungsmittel können verwendet werden.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I können außerdem durch Umsetzen der Verbindungen der Formel II mit Hydroxylaminen der Formel $R_2O$-$NH_2$, in der $R^2$ die obengenannten Bedeutungen hat, in inerten Verdünnungsmitteln bei einer Temperatur zwischen 0°C und dem Siedepunkt des Reaktionsgemisches, üblicherweise zwischen 0 und 80°C, vorzugsweise zwischen 15 und 70°C, erhalten werden. Gegebenenfalls kann das Hydroxylamin als wäßrige Lösung eingesetzt werden.

Sie können ferner erhalten werden durch Umsetzung der Verbindung mit der Formel II mit unsubstituiertem Hydroxylammoniumsalz $NH_2OH$ . HY, wobei Y ein beliebiges Anion bedeutet, bei einer Temperatur zwischen 0 und 80°C in Gegenwert eines Lösungsmittels und einer Base und Alkylierung des erhaltenen Oxims der Formel

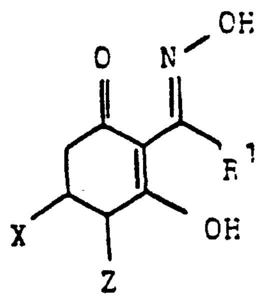

(III)

in der $R^1$, X und Z die in Anspruch 1 genannten Bedeutungen haben, mit einem Alkylierungsmittel der Formel

$R^2$-Y' (IV),

in der Y' eine Abgangsgruppe bedeutet und $R^2$ die in Anspruch 1 genannten Bedeutungen hat.

Geeignete Lösungsmittel für diese Umsetzungen sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran. Auch Gemische dieser Lösungsmittel können verwendet werden.

Die Alkalimetallsalze der Cyclohexan-1,3-dionderivate der Formel I können durch Behandeln dieser Verbindungen mit Natrium- oder Kaliumhydroxid in wäßriger Lösung oder in einem organischen Lösungsmittel, wie Methanol, Ethanol, Aceton, erhalten werden. Auch Natrium- und Kaliumalkoholate können als Basen dienen.

Die anderen Metallsalze, z. B. die Mangan-, Kupfer-, Zink-, Eisen-, Calcium-, Magnesium- und Bariumsalze können aus den Natriumsalzen durch Reaktion mit den entsprechenden Metallchloriden in wäßriger Lösung hergestellt werden. Ammonium-, Phosphonium-, Sulfonium- oder Sulfoxoniumsalze können durch Umsetzung von Verbindungen der Formel 1 mit Ammonium-, Phosphonium-, Sulfonium- oder Sulfoxoniumhydroxiden gegebenenfalls in wäßriger Lösung hergestellt werden.

Die Verbindungen der Formel II können aus Cyclohexan-1,3-dionen der Formel IV, die auch in den tautomeren Formeln IV a und IV b vorliegen können,

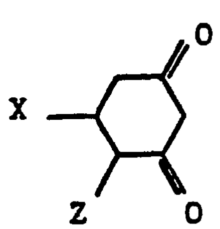

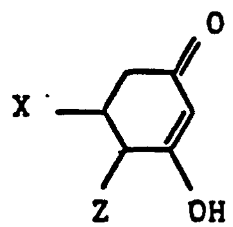

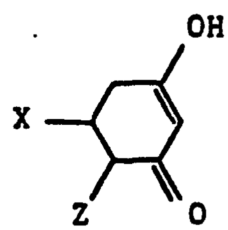

IV IVa IVb

nach literaturbekannten Methoden (Tetrahedron Letters, 29, 2491 (1975)) hergestellt werden.

Es ist auch möglich, Verbindungen der Formel II über die Zwischenstufe der Enolester, die bei der Umsetzung von Verbindungen der Formel IV eventuell als Isomerengemische anfallen und in Gegenwart von Imidazol- oder Pyridinderivaten umgelagert werden (JP-OS 79/063052), herzustellen.

Zu den Verbindungen der Formel IV gelangt man nach literaturbekannten Verfahren, wie dies aus folgendem Schema hervorgeht:

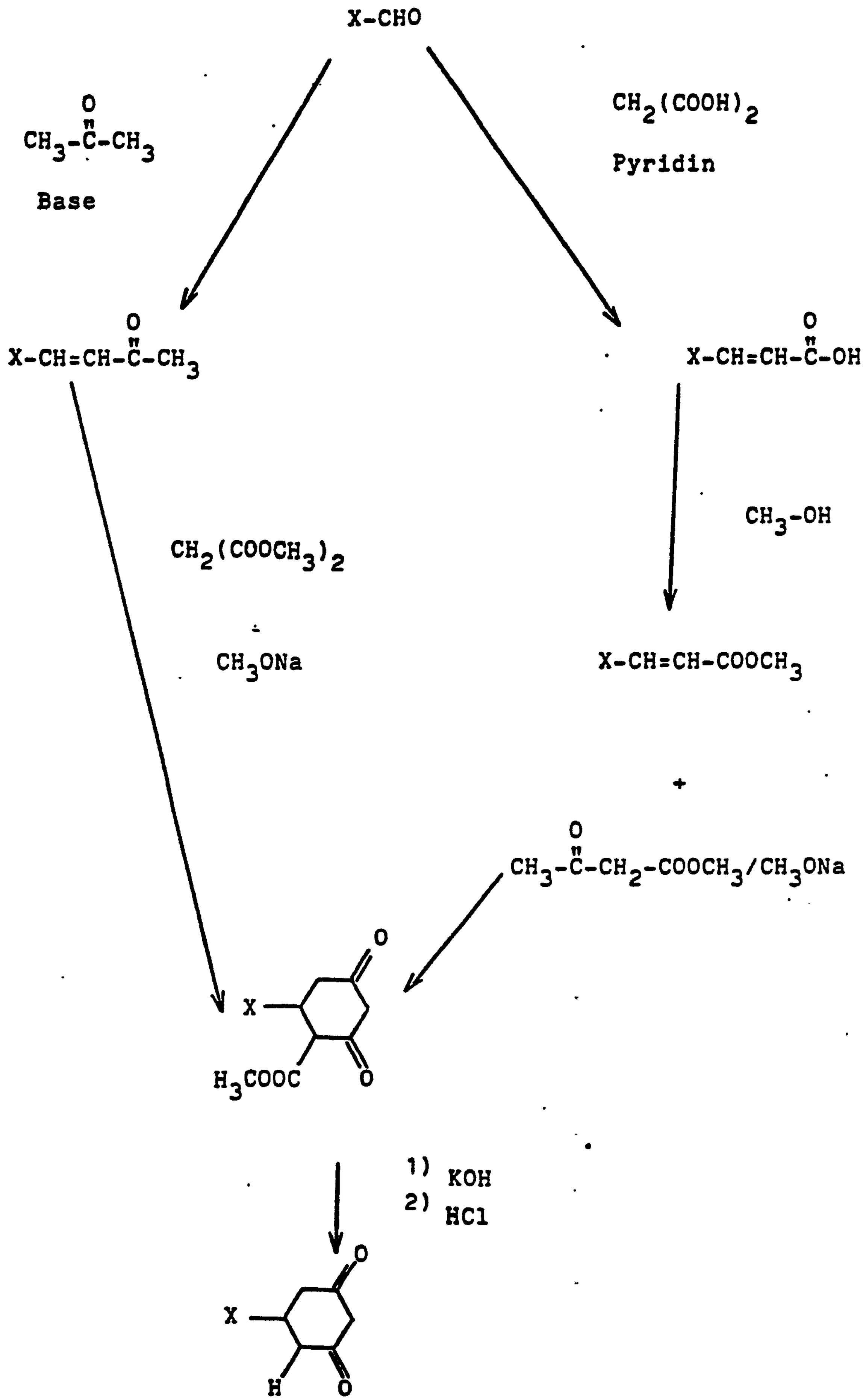
Base
CH2(COOH)2
Pyridin
CH3-OH
CH2(COOCH3)2
CH3ONa
1) KOH
2) HCl

Das folgende Beispiel erläutert die Herstellung der erfindungsgemäßen Cyclohexan-1,3-dionderivate der Formel I. Dabei verhalten sich Gewichtsteile zu Volumenteilen wie Kilogramm zu Liter.

**Bespiel 1**

10,2 Gewichtsteile 2-Butyryl-5-(3-methyl-4-ethyl-6-isobutoxytetrahydro-pyran-2-yl)-cyclohexan-1,3-dion werden in 100 Volumenteilen Ethanol gelöst und mit 2,42 Gewichtsteilen Natriumacetat sowie 3,86 Gewichtsteilen 0-(3-Chlorallyl)-hydroxylaminhydrochlorid versetzt. Mach mehrstündigem Rühren bei Raumtemperatur wird in Eiswasser gegeben und mit Methylenchlorid extrahiert. Mach Abdampfen der Methylenchloridphase verbleiben 11,1 Gewichtsteile 2-(3-Chlorallyloxyaminobutyliden)-5-(3-methyl-4-ethyl-6-isobutoxytetrahydropyran-2-yl)-cyclohexan-1,3-dion als gelbes Öl mit dem Brechungsindex $n_D^{22}$: 1,5129.

Die folgenden Substanzen lassen sich auf analogem Wege herstellen:

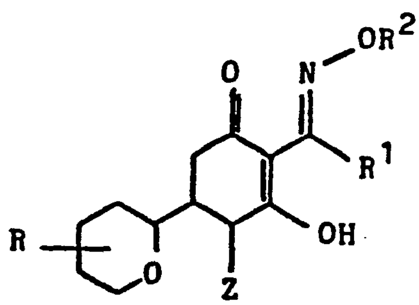

| Nr. | R | $R^1$ | $R^2$ | Z | $n_D$/Fp [°C] |
|---|---|---|---|---|---|
| 1 | 3-$CH_3$, 4-$C_2H_5$, 6-$OCH_2CH(CH_3)_2$ | $C_3H_7$ | $CH_2CH=CHCl$ | H | $n_D^{22}=1{,}5129$ |
| 2 | 3-$CH_3$, 4-$C_2H_5$, 6-$OCH_2CH(CH_3)_2$ | $C_3H_7$ | $C_2H_5$ | H | $n_D^{22}=1{,}5006$ |
| 3 | 3-$CH_3$, 4-$C_2H_5$, 6-$OCH_2CH(CH_3)_2$ | $C_3H_7$ | $CH_2CH=CH_2$ | $COOCH_3$ | $n_D^{23}=1{,}5015$ |
| 4 | 6-$OCH_3$ | $C_2H_5$ | $C_2H_5$ | H | $n_D^{27}=1{,}5158$ |
| 5 | 4-$C_6H_5$, 6-$OCH_3$ | $C_2H_5$ | $C_2H_5$ | H | |
| 6 | 6-$OCH_3$ | $C_3H_7$ | $C_2H_5$ | H | Fp. 69-71 |
| 7 | 3-$CH_3$, 4-$C_2H_5$, 6-$OCH_2CH(CH_3)_2$ | $C_2H_5$ | $C_2H_5$ | H | |
| 8 | 4-$C_6H_5$, 6-$OCH_3$ | $C_3H_7$ | $C_2H_5$ | H | |
| 9 | 3-$CH_3$, 4-$C_2H_5$, 6-$OCH_2(CH_3)_2$ | $C_3H_7$ | $CH_2CH=CH_2$ | H | $n_D^{22}=1{,}5052$ |
| 10 | 6-$OCH_3$ | $C_3H_7$ | $C_2H_5$ | $COOCH_3$ | $n_D^{26}=1{,}5050$ |
| 11 | 3-$C_2H_5$, 4-$C_3H_7$, 6-$OCH_3$ | $C_2H_5$ | $C_2H_5$ | H | |
| 12 | 4-$C_6H_5$, 6-$OCH_3$ | $C_3H_7$ | $CH_2CH=CH_2$ | H | |
| 13 | 3-$CH_3$, 4-$C_2H_5$, 6-$OCH_2CH(CH_3)_2$ | $C_2H_5$ | $CH_2CH=CH_2$ | H | |
| 14 | 6-$OCH_3$ | $C_2H_5$ | $CH_2CH=CH_2$ | H | $n_D^{25}=1{,}5227$ |
| 15 | 4-$C_6H_5$, 6-$OCH_3$ | $C_2H_5$ | $CH_2CH=CH_2$ | H | |
| 16 | 3-$C_2H_5$, 4-$C_3H_7$, 6-$OCH_3$ | $C_2H_5$ | $CH_2CH=CH_2$ | H | |
| 17 | 6-$OCH_3$ | $C_3H_7$ | $CH_2CH=CH_2$ | H | $n_D^{26}=1{,}5164$ |
| 18 | 3-$C_2H_5$, 4-$C_3H_7$, 6-$OCH_3$ | $C_3H_7$ | $C_2H_5$ | H | $n_D^{23}=1{,}5080$ |
| 19 | 6-$OCH_3$ | $C_2H_5$ | $CH_2CH=CHCl$ | H | |
| 20 | 3-$C_2H_5$, 4-$C_3H_7$, 6-$OCH_3$ | $C_3H_7$ | $CH_2CH=CH_2$ | H | $n_D^{23}=1{,}5133$ |
| 21 | 3-$CH_3$-, 6-$OCH_3$ | $C_3H_7$ | $C_2H_5$ | H | |
| 22 | 6-$OCH_3$ | $C_3H_7$ | $CH_2CH=CH_2$ | $COOCH_3$ | $n_D^{30}=1{,}5077$ |
| 23 | 3-$CH_3$, 6-$OCH_3$ | $C_2H_5$ | $CH_2CH=CH_2$ | H | |
| 24 | 6-$OCH(CH_3)_2$ | $C_2H_5$ | $C_2H_5$ | H | |
| 25 | 6-$OCH(CH_3)2$ | $C_2H_5$ | $CH_2CH=CH_2$ | H | |
| 26 | 3-$CH_3$, 6-$OCH_3$ | $C_3H_7$ | $CH_2CH=CH_2$ | H | |
| 27 | 3-$CH_3$, 6-$OCH_3$ | $C_3H_7$ | $C_2H_5$ | H | |
| 28 | 3-$CH_3$, 4-$C_2H_5$, 6-$OCH_3$ | $C_3H_7$ | $C_2H_5$ | H | |
| 29 | 3-$CH_3$, 6-$OC_2H_5$ | $C_3H_7$ | $C_2H_5$ | H | |
| 30 | 6-$OC_2H_5$ | $C_2H_5$ | $C_2H_5$ | H | |
| 31 | 3-$CH_3$, 4-$C_2H_5$, 6-$OCH(CH_3)_2$ | $C_3H_7$ | $C_2H_5$ | H | |
| 32 | 3$CH_3$, 4-$C_2H_5$, 6-$OCH_3$ | $C_2H_5$ | $C_2H_5$ | H | |
| 33 | 3-$CH_3$ 6-$OC_2H_5$ | $C_3H_7$ | $CH_2CH=CH_2$ | H | |
| 34 | 4-$C_6H_5$, 6-$OCH(CH_3)_2$ | $C_2H_5$ | $C_2H_5$ | H | |

| | | | | | |
|---|---|---|---|---|---|
| 35 | 3-$CH_3$, 4-$C_2H_5$, 6-$OCH_3$ | $C_2H_5$ | $CH_2CH=CH_2$ | H | |
| 36 | 4-$C_6H_5$, 6-$OCH(CH_3)_2$ | $C_3H_7$ | $C_2H_5$ | H | |
| 37 | 3-$CH_3$, 4-$C_2H_5$, 6-$OC_2H_5$ | $C_3H_7$ | $C_2H_5$ | H | |
| 38 | 6-$OC_2H_5$ | $C_3H_7$ | $C_2H_5$ | H | $n_D^{21}=1,5090$ |
| 39 | 3-$CH_3$, 6-$OC_2H_5$ | $C_2H_5$ | $CH_2CH=CH_2$ | H | |
| 40 | 3-$CH_3$, 4-$C_2H_5$, 6-$OCH_3$ | $C_3H_7$ | $CH_2CH=CH_2$ | H | |
| 41 | 4-$C_6H_5$, 6-$OCH(CH_3)_2$ | $C_3H_7$ | $CH_2CH=CH_2$ | H | |
| 42 | 4-$C_6H_5$, 6-$OCH(CH_3)_2$ | $C_2H_5$ | $C_2CH=CH_2$ | H | |
| 43 | 6-$OC_2H_5$ | $C_3H_7$ | $CH_2CH=CH_2$ | H | $n_D^{22}=1,5150$ |
| 44 | 6-$OC_2H_5$ | $C_2H_5$ | $CH_2CH=CH_2$ | H | |
| 45 | 3-$CH_3$, 6-$OC_2H_5$ | $C_2H_5$ | $C_2H_5$ | H | |
| 46 | 6-$OCH(CH_3)_2$ | $C_3H_7$ | $C_2H_5$ | H | |
| 47 | 6-$OCH(CH_3)_2$ | $C_3H_7$ | $CH_2=CH_2$ | H | |
| 48 | 3-$C_2H_5$, 4-$C_3H_7$, 6-$OC_2H_5$ | $C_3H_7$ | $C_2H_5$ | H | |
| 49 | 3-$C_2H_5$, 4-$C_3H_7$, 6-$OCH(CH_3)_2$ | $C_3H_7$ | $C_2H_5$ | H | |
| 50 | 4-$C_6H_5$, 6-$OC_2H_5$ | $C_3H_7$ | $C_2H_5$ | H | |
| 51 | 3-$C_2H_5$, 4-$C_3H_7$, 6-$OCH_2CH(CH_3)_2$ | $C_3H_7$ | $C_2H_5$ | H | |
| 52 | 3-$CH_3$, 6-$OCH(CH_3)_2$ | $C_2H_5$ | $C_2H_5$ | H | |
| 53 | 3-$CH_3$, 6-$OCH(CH_3)_2$ | $C_3H_7$ | $C_2H_5$ | H | |
| 54 | 3-$CH_3$, 6-$OCH(CH_3)_2$ | $C_2H_5$ | $CH_2CH=CH_2$ | H | |
| 55 | 6-$OC_6H_5$ | $C_3H_7$ | $C_2H_5$ | H | |
| 56 | 3-$C_2H_5$, 4-$C_3H_7$, 6-$OCH_2CH(CH_3)_2$ | $C_3H_7$ | $CH_2CH=CH_2$ | H | |
| 57 | 3-$CH_3$ 6-$OCH(CH_3)_2$ | $C_3H_7$ | $CH_2CH=CH_2$ | H | |
| 58 | 6-$OC_6H_5$ | $C_3H_7$ | $CH_2CH=CH_2$ | H | |
| 59 | 6-$OC_6H_5$ | $C_2H_5$ | $C_2H_5$ | H | |
| 60 | 6-$OC_6H_5$ | $C_2H_5$ | $CH_2CH=CH_2$ | H | |
| 61 | 3-$C_2H_5$, 4-$C_3H_7$, 6-$OCH_2CH(CH_3)_2$ | $C_2H_5$ | $C_2H_5$ | H | |
| 62 | 3-$C_2H_5$, 4-$C_3H_7$, 6-$OCH_2CH(CH_3)_2$ | $C_2H_5$ | $CH_2CH=CH_2$ | H | |
| 63 | 4-$C_6H_5$, 6-$OC_2H_5$ | $C_2H_5$ | $C_2H_5$ | H | |
| 64 | 6-$OCH_2CH(CH_3)_2$ | $C_2H_5$ | $C_2H_5$ | H | |
| 65 | 3-$CH_3$, 6-$OCH_2CH(CH_3)_2$ | $C_2H_5$ | $C_2H_5$ | H | |
| 66 | 6-$OCH_2CH(CH_3)_2$ | $C_3H_7$ | $C_2H_5$ | H | |
| 67 | 3-$CH_3$, 6-$OCH_2CH(CH_3)_2$ | $C_3H_7$ | $C_2H_5$ | H | |
| 68 | 6-$OCH_2C_6H_5$ | $C_3H_7$ | $C_2H_5$ | H | |
| 69 | 6-$OCH_2C_6H_5$ | $C_3H_7$ | $CH_2CH=CH_2$ | H | |
| 70 | 4-$C_6H_5$, 6-$OCH_2CH(CH_3)_2$ | $C_2H_5$ | $C_2H_5$ | H | |
| 71 | 4-$C_6H_5$, 6-$OC_6H_4Cl$ | $C_3H_7$ | $C_2H_5$ | H | |
| 72 | 4-$C_6H_5$, 6-OC6$H_4Cl$ | $C_3H_7$ | $CH_2CH=CH_2$ | H | |
| 73 | 6-$SCH_3$ | $C_2H_5$ | $C_2H_5$ | H | |
| 74 | 6-$SC_2H_5$ | $C_2H_5$ | $C_2H_5$ | H | |
| 75 | 6-$SCH_3$ | $C_2H_5$ | $CH_2CH=CH_2$ | H | |
| 76 | 6-$SCH_3$ | $C_3H_7$ | $CH_2CH=CH_2$ | H | |
| 77 | 6-$SCH_3$ | $C_3H_7$ | $C_2H_5$ | H | |
| 78 | 6-$SC_2H_5$ | $C_3H_7$ | $C_2H_5$ | H | |
| 79 | 6-$SC_2H_5$ | $C_3H_7$ | $CH_2CH=CH_2$ | H | |
| 80 | 6-$SC_2H_5$ | $C_2H_5$ | $CH_2CH=CH_2$ | H | |

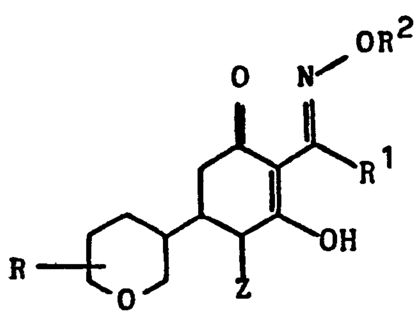

| | | | | |
|---|---|---|---|---|
| 81 | 6-$OCH_3$ | $C_2H_5$ | $C_2H_5$ | H |
| 82 | 6-$SCH_3$ | $C_2H_5$ | $CH_2CH=CH_2$ | H |
| 83 | 6-$SCH_3$ | $C_2H_5$ | $C_2H_5$ | H |
| 84 | 2-$CH_3$, 6-$OCH_3$ | $C_2H_5$ | $C_2H_5$ | H |
| 85 | 2-$CH_3$, 6-$OC_2H_5$ | $C_2H_5$ | $C_2H_5$ | H |

| | | | | | |
|---|---|---|---|---|---|
| 86 | 6-$CH_3$ | $C_3H_7$ | $C_2H_5$ | H | |
| 87 | 2-$CH_3$, 6-$OCH_3$ | $C_2H_5$ | $CH_2CH=CH_2$ | H | |
| 88 | 2-$CH_3$, 6-$OC_2H_5$ | $C_2H_5$ | $CH_2CH=CH_2$ | H | |
| 89 | 6-$SCH_3$ | $C_3H_7$ | $CH_2CH=CH_2$ | H | |
| 90 | 6-$OCH_3$ | $C_3H_7$ | $C_2H_5$ | H | $n_D^{26}=1,5163$ |
| 91 | 2-$CH_3$, 6-$OCH_3$ | $C_3H_7$ | $CH_2CH=CH_2$ | H | |
| 92 | 2-$CH_3$, 6-$OCH_3$ | $C_3H_7$ | $C_2H_5$ | H | |
| 93 | 2-$CH_3$, 6-$OC_2H_5$ | $C_3H_7$ | $CH_2CH=CH_2$ | H | |
| 94 | 6-$OCH_3$ | $C_3H_7$ | $CH_2CH=CH_2$ | H | $n_D^{25}=1,5219$ |
| 95 | 6-$OCH_3$ | $C_2H_5$ | $CH_2CH=CH_2$ | H | |
| 96 | 2-$CH_3$, 6-$OC_2H_5$ | $C_3H_7$ | $C_2H_5$ | H | |
| 97 | 2-$CH_3$, 6-$OCH_2CH(CH_3)_2$ | $C_2H_5$ | $C_2H_5$ | H | |
| 98 | 2-$CH_3$, 6-$OCH_2CH(CH_3)_2$ | $C_3H_7$ | $C_2H_5$ | H | |
| 99 | 2-$CH_3$, 6-$OCH(CH_3)_2$ | $C_2H_5$ | $C_2H_5$ | H | |
| 100 | 2-$CH_3$, 6-$OCH_2CH(CH_3)_2$ | $C_2H_5$ | $CH_2CH=CH_2$ | H | |
| 101 | 2-$CH_3$, 6-$OCH(CH_3)_2$ | $C_2H_5$ | $CH_2CH=CH_2$ | H | |
| 102 | 2-$CH_3$, 6-$OCH_2CH(CH_3)_2$ | $C_3H_7$ | $CH_2CH=CH_2$ | H | |
| 103 | 2-$CH_3$, 6-$OC_6H_5$ | $C_2H_5$ | $C_2H_5$ | H | |
| 104 | 6-$OC_2H_5$ | $C_2H_5$ | $C_2H_5$ | H | |
| 105 | 2-$CH_3$, 6-$OC_6H_5$ | $C_2H_5$ | $CH_2CH=CH_2$ | H | |
| 106 | 6-$OC_2H_5$ | $C_2H_5$ | $CH_2CH=CH_2$ | H | |
| 107 | 6-$OC_2H_5$ | $C_3H_7$ | $CH_2CH=CHCl$ | H | |
| 108 | 2-$CH_3$, 6-$OC_6H_5$ | $C_3H_7$ | $C_2H_5$ | H | |
| 109 | 2-$CH_3$, 6-$OC_6H_5$ | $C_3H_7$ | $CH_2CH=CH_2$ | H | |
| 110 | 6-$SC_2H_5$ | $C_3H_7$ | $C_2H_5$ | H | |
| 111 | 6-$SC_2H_5$ | $C_3H_7$ | $CH_2CH=CH_2$ | H | |
| 112 | 8-$OC_2H_5$ | $C_3H_7$ | $C_2H_5$ | H | |
| 113 | 6-$OCH_3$ | $C_3H_7$ | $CH_2$-C=CH | H | |
| 114 | 6-$OC_2H_5$ | $C_3H_7$ | $CH_2CH=CH_2$ | H | |
| 115 | 6-$SC_2H_5$ | $C_2H_5$ | $C_2H_5$ | H | |
| 116 | 6-$OCH_3$ | $C_3H_7$ | $CH_2CH=CHCl$ | H | $n_D^{31}=1,5317$ |
| 117 | 6-$OCH_3$ | $C_3H_7$ | $C_3H_7$ | H | $n_D^{31}=1,5125$ |
| 118 | 6-$SC_2H_5$ | $C_2H_5$ | $CH_2CH=CH_2$ | H | |
| 119 | 2-$CH_3$, 4-$C_6H_5$, 6-$OCH_3$ | $C_3H_7$ | $CH_2CH=CH_2$ | H | |
| 120 | 2-$CH_3$, 4-$C_6H_5$, 6-$OC_2H_5$ | $C_3H_7$ | $C_2H_5$ | H | |
| 121 | 2-$CH_3$, 4-$C_6H_5$, 6-$OCH_3$ | $C_3H_7$ | $C_2H_5$ | H | |
| 122 | 2-$CH_3$, 4-$C_6H_5$, 6-$OCH_2CH(_3)_2$ | $C_3H_7$ | $CH_2CH=CH_2$ | H | |
| 123 | 2-$CH_3$, 4-$C_6H_5$, 6-$OCH_2CH(_3)_2$ | $C_3H_7$ | $C_2H_5$ | H | |
| 124 | 2-$CH_3$, 4-$C_6H_5$, 6-$OC_2H_5$ | $C_3H_7$ | $CH_2CH=CH_2$ | H | |
| 125 | 2-$OCH_3$ | $C_3H_7$ | $C_2H_5$ | H | $n_D^{28}=1,5135$ |
| 126 | 2-$OC_2H_5$ | $C_3H_7$ | $CH_2CH=CH_2$ | H | |
| 127 | 2-$OCH_3$ | $C_3H_7$ | $CH_2CH=CH_2$ | H | |
| 128 | 2-$OCH_3$ | $C_2H_5$ | $C_2H_5$ | H | |
| 129 | 2-$OC_2H_5$ | $C_2H_5$ | $C_2H_5$ | H | |
| 130 | 2-$OC_2H_5$ | $C_2H_5$ | $CH_2CH=CH_2$ | H | |
| 131 | 2-$SCH_3$ | $C_3H_7$ | $C_2H_5$ | H | |
| 132 | 2-$OCH_3$ | $C_2H_5$ | $CH_2CH=CH_2$ | H | |
| 133 | 2-$SCH_3$ | $C_3H_7$ | $CH_2CH=CH_2$ | H | |
| 134 | 2-$SC_2H_5$ | $C_2H_5$ | $C_2H_5$ | H | |
| 135 | 2-$OC_2H_5$ | $C_3H_7$ | $C_2H_5$ | H | |
| 136 | 2-$SCH_3$ | $C_2H_5$ | $CH_2CH=CH_2$ | H | |
| 137 | 2-$SC_2H_5$ | $C_3H_7$ | $C_2H_5$ | H | |
| 138 | 2-$SC_2H_5$ | $C_2H_5$ | $CH_2CH=CH_2$ | H | |
| 139 | 2-$SC_2H_5$ | $C_3H_7$ | $CH_2CH=CH_2$ | H | |
| 140 | 2-$SCH_3$ | $C_2H_5$ | $C_2H_5$ | H | |
| 141 | 2-$OCH(CH_3)_2$ | $C_3H_7$ | $C_2H_5$ | H | |
| 142 | 2-$OCH_2CH(CH_3)_2$ | $C_3H_7$ | $C_2H_5$ | H | |
| 143 | 2-$OC_6H_5$ | $C_2H_5$ | $C_2H_5$ | H | |
| 144 | 2-$OC_6H_5$ | $C_3H_7$ | $C_2H_5$ | H | |
| 145 | 2-$OC_6H_5$ | $C_2H_5$ | $CH_2CH=CH_2$ | H | |
| 146 | 2-$OC_6H_5$ | $C_3H_7$ | $CH_2CH=CH_2$ | H | |
| 147 | 6-$OCH(CH_3)_2$ | $C_2H_5$ | $C_2H_5$ | H | |
| 148 | 6-$OCH_2CH(CH_3)_2$ | $C_2H_5$ | $C_2H_5$ | H | |
| 149 | 6-$OC_6H_5$ | $C_2H_5$ | $C_2H_5$ | H | |
| 150 | 6-$OC_2H_5$ | $C_2H_5$ | $CH_2CH=CH_2$ | H | |

| | | | | | |
|---|---|---|---|---|---|
| 151 | 6-$OC_6H_5$ | $C_3H_7$ | $C_2H_5$ | H | |
| 152 | 6-$OC_6H_5$ | $C_3H_7$ | $CH_2CH=CH_2$ | H | |
| 153 | 4-$SC_2H_5$ | $C_3H_7$ | $C_2H_5$ | H | $n_D^{19}=1{,}5428$ |
| 154 | 4-$OCH_3$ | $C_2H_5$ | $C_2H_5$ | H | |
| 155 | 4-$SC_2H_5$ | $C_3H_7$ | $CH_2CH=CH_2$ | H | $n_D^{19}=1{,}5481$ |
| 156 | 4-$OCH_3$ | $C_3H_7$ | $C_2H_5$ | H | |
| 157 | 4-$OC_2H_5$ | $C_2H_5$ | $C_2H_5$ | H | |
| 158 | 4-$SCH_3$ | $C_2H_5$ | $C_2H_5$ | H | |
| 159 | 4-$OCH_3$ | $C_3H_7$ | $CH_2CH=CH_2$ | H | |
| 160 | 4-$OCH_3$ | $C_2H_5$ | $CH_2CH=CH_2$ | H | |
| 161 | 4-$SC_2H_5$ | $C_2H_5$ | $C_2H_5$ | H | |
| 162 | 4-$SC_2H_5$ | $C_2H_5$ | $CH_2CH=CH_2$ | H | |
| 163 | 4-$OC_2H_5$ | $C_2H_5$ | $CH_2CH=CH_2$ | H | |
| 164 | 4-$OC_2H_5$ | $C_3H_7$ | $C_2H_5$ | H | |
| 165 | 4-$SCH_3$ | $C_2H_5$ | $CH_2CH=CH_2$ | H | |
| 1166 | 4-$OCH(CH_3)_2$ | $C_3H_7$ | $CH_2CH=CH_2$ | H | |
| 167 | 4-$OCH_2H_5$ | $C_3H_7$ | $CH_2CH=CH_2$ | H | |
| 168 | 4-$OCH_2CH(CH_3)_2$ | $C_3H_7$ | $C_2H_5$ | H | |
| 169 | 4-$SCH_3$ | $C_3H_7$ | $CH_2CH=CH_2$ | H | |
| 170 | 2-$CH_3$, 4-$C_6H_5$, 6-$OCH(CH_3)_2$ | $C_3H_7$ | $C_2H_5$ | H | |
| 171 | 2-$CH_3$, 4-$C_6H_5$, 6-$OCH(CH_3)_2$ | $C_2H_5$ | $C_2H_5$ | H | |
| 172 | 2-$CH_3$, 4-$C_6H_5$, 6-$OCH(CH_3)_2$ | $C_3H_7$ | $CH_2CH=CH_2$ | H | |
| 173 | 4-$SCH_3$ | $C_3H_7$ | $C_2H_5$ | H | |
| 174 | 4-$SC_6H_5$ | $C_3H_7$ | $C_2H_5$ | H | |
| 175 | 4-$OC_6H_5$ | $C_2H_5$ | $C_2H_5$ | H | |

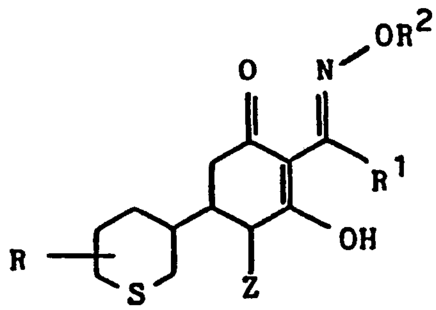

| | | | | |
|---|---|---|---|---|
| 176 | 2-$OCH_3$ | $C_2H_5$ | $C_2H_5$ | H |
| 177 | 2-$SCH_3$ | $C_2H_5$ | $CH_2CH=CH_2$ | H |
| 178 | 2-$SCH_3$ | $C_3H_7$ | $CH_2CH=CH_2$ | H |
| 179 | 2-$OCH_3$ | $C_2H_5$ | $CH_2CH=CH_2$ | H |
| 180 | 2-$OCH_3$ | $C_3H_7$ | $C_2H_5$ | H |
| 181 | 2-$OC_2H_5$ | $C_2H_5$ | $C_2H_5$ | H |
| 182 | 2-$OCH_3$ | $C_3H_7$ | $CH_2CH=CH_2$ | H |
| 183 | 2-$OC_2H_5$ | $C_2H_5$ | $CH_2CH=CH_2$ | H |
| 184 | 2-$SC_2H_5$ | $C_2H_5$ | $C_2H_5$ | H |
| 185 | 2-$SCH_3$ | $C_2H_5$ | $C_2H_5$ | H |
| 186 | 2-$SCH_3$ | $C_3H_7$ | $C_2H_5$ | H |
| 187 | 4-$SCH_3$ | $C_2H_5$ | $C_2H_5$ | H |
| 188 | 2-$SC_2H_5$ | $C_3H_7$ | $CH_2CH=CH_2$ | H |
| 189 | 4-$SCH_3$ | $C_2H_5$ | $CH_2CH=CH_2$ | H |
| 190 | 2-$OCH(CH_3)_2$ | $C_2H_5$ | $C_2H_5$ | H |
| 191 | 2-$OC_6H_5$ | $C_2H_5$ | $C_2H_5$ | H |
| 192 | 2-$OC_6H_5$ | $C_2H_5$ | $C_2H_5$ | H |
| 193 | 4-$SCH_3$ | $C_3H_7$ | $C_2H_5$ | H |
| 194 | 4-$SCH_3$ | $C_3H_7$ | $CH_2CH=CH_2$ | H |
| 195 | 4-$OC_2H_5$ | $C_2H_5$ | $C_2H_5$ | H |
| 196 | 4-$OC_2H_5$ | $C_2H_5$ | $CH_2CH=CH_2$ | H |
| 197 | 4-$OCH_3$ | $C_3H_7$ | $CH_2CH=CH_2$ | H |
| 198 | 4-$OCH_3$ | $C_3H_7$ | $C_2H_5$ | H |
| 199 | 4-$SC_2H_5$ | $C_2H_5$ | $C_2H_5$ | H |
| 200 | 4-$SC_2H_5$ | $C_2H_5$ | $CH_2CH=CH_2$ | H |
| 201 | 4-$OCH_3$ | $C_2H_5$ | $C_2H_5$ | H |

| | | | | | |
|---|---|---|---|---|---|
| 202 | 4-$SC_2H_5$ | $C_3H_7$ | $C_2H_5$ | H | $n_D^{27} = 1,5637$ |
| 203 | 4-$SC_2H_5$ | $C_3H_7$ | $CH_2CH = CH_2$ | H | $n_D^{22} = 1,5672$ |
| 204 | 4-$OCH_3$ | $C_2H_5$ | $CH_2CH = CH_2$ | H | |
| 205 | 4-$SC_6H_5$ | $C_3H_7$ | $C_2H_5$ | H | |
| 206 | 4-$OC_6H_5$ | $C_3H_7$ | $C_2H_5$ | H | |

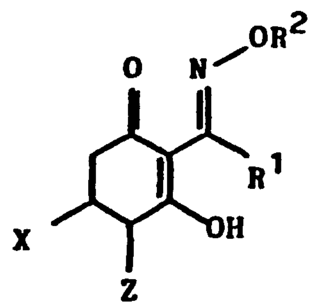

| | | | | | |
|---|---|---|---|---|---|
| 207 | 2,5-Dimethoxytetrahydrofuran-3-yl | $C_2H_5$ | $C_2H_5$ | H | |
| 208 | 2,5-Dimethoxytetrahydrofuran-3-yl | $C_2M_5$ | $CH_2CH = CH_2$ | H | |
| 209 | 2,5-Dimethoxytetrahydrofuran-3-yl | $C_3H_7$ | $C_2H_5$ | H | |
| 210 | 2,5-Dimethoxytetrahydrofuran-3-yl | $C_3H_7$ | $CH_2CH = CH_2$ | H | |
| 211 | 2-Methoxy-1,4-dioxan-3-yl | $C_3H_7$ | $C_2H_5$ | H | |
| 212 | 2-Methoxy-1,4-dioxan-3-yl | $C_3H_7$ | $CH_2CH = CH_2$ | H | |
| 213 | 3-Ethyl-6-isobutoxytetrahydro--pyran-2-yl | $C_3H_7$ | $C_2H_5$ | H | |
| 214 | 3-Ethyl-6-isobutoxytetrahydro--pyran-2-yl | $C_3H_7$ | $CH_2CH = CH_2$ | H | |
| 215 | 6-Cyclohexoxytetrahydropyran-2-yl | $C_3H_7$ | $C_2H_5$ | $COOCH_3$ | $n_D^{25} = 1,5093$ |
| 216 | 6-Cyclohexoxytetrahydropyran-2-yl | $C_3H_7$ | $CH_2CH = CH_2$ | $COOCH_3$ | $n_D^{24} = 1,5138$ |
| 217 | 3-Methyl-6-isobutoxytetrahydro-pyran-2-yl- | $C_3H_7$ | $C_2H_5$ | $COOCH_3$ | $n_D^{25} = 1,5002$ |
| 218 | 3-Methyl-6-isobutoxytetrahydro-pyran-2-yl- | $C_3H_7$ | $CH_2CH = CH_2$ | $COOCH_3$ | $N_D^{26} = 1,4988$ |
| 219 | 3-Methyl-6-isobutoxytetrahydro-pyran-2-yl- | $C_3H_5$ | $C_2H_5$ | $COOCH_3$ | $n_D^{25} = 1,5007$ |
| 220 | 3-Methyl-6-isobutoxytetrahydro-pyran-2-yl- | $C_2H_5$ | $CH_2CH = CH_2$ | $COOCH_3$ | $n_D^{24} = 1,5060$ |
| 221 | 3-Methyl-6-benzoxytetrahydro-pyran-2-yl | $C_3H_7$ | $C_2H_5$ | $COOCH_3$ | $n_D^{24} = 1,5294$ |
| 222 | 3-Methyl-6-benzoxytetrahydro-pyran-2-yl | $C_3H_7$ | $CH_2CH = CH_2$ | $COOCH_3$ | $n_D^{24} = 1,5352$ |
| 223 | 6-Benzoxytetrahydropyran-2-yl | $C_3H_7$ | $C_2H_5$ | $COOCH_3$ | $n_D^{26} = 1,5303$ |
| 224 | 6-Benzoxytetrahydropyran-2-yl | $C_3H_7$ | $CH_2CH = CH_2$ | $COOCH_3$ | $n_D^{24} = 1,5382$ |
| 225 | 6-Benzoxytetrahydropyran-2-yl | $C_3H_7$ | $C_2H_5$ | H | $n_D^{23} = 1,5418$ |
| 226 | 6-Benzoxytetrahydropyran-2-yl | $C_3H_7$ | $CH_2CH = CH_2$ | H | $n_D^{23} = 1,5462$ |
| 227 | 3-Methyl-6-isobutoxytetra-hydropyran-2-yl | $C_3H_7$ | $C_2H_5$ | H | |
| 228 | 3-Methyl-6-isobutoxytetra-hydropyran-2-yl | $C_3H_7$ | $CH_2CH = CH_2$ | H | $n_D^{24} = 1,5047$ |
| 229 | 3-Methyl-6-isobutoxytetra-hydropyran-2-yl | $C_2H_5$ | $C_2H_5$ | H | $n_D^{24} = 1,5033$ |
| 230 | 3-Methyl-6-isobutoxytetra-hydropyran-2-yl | $C_2H_5$ | $CH_2CH = CH_2$ | H | $n_D^{24} = 1,5078$ |
| 231 | 6-Cyclohexoxytetrahydropyran-2-yl | $C_3H_7$ | $C_2H_5$ | H | |
| 232 | 6-Cyclohexoxytetrahydropyran-2-yl | $C_3H_7$ | $CH_2CH = CH_2$ | H | $n_D^{24} = 1,5218$ |

Die Cyclohexan-1,3-dionderivate der Formel I und ihre Salze können bei-Spielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen

Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stsrk polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthelinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Mußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

**Beispiele für Formulierungen sind:**

I. Man vermischt 90 Gewichtsteile der Verbindung Nr. 2 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 9 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 17 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der lösung in 100 000 Gewichtsteilen Wasser erhält man eine wäßrige Dispersion, die 0,02 Gewichtsprozent des Wirkstoffs enthält.

IV. 20 Gewichtsteile des Wirkstoffs Nr. 6 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile des Wirkstoffs Nr. 116 werden mit 3 Gewichtsteilen des Natriumsalzes der Diisobutylnaphthalin- -sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 117 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 169 werden mit einer Mischung aus 92 Gewichtsteilen pulverförmigem Kieselsäuregel und 8 Gewichtsteilen Paraffinöl, das auf die Oberfläche dieses Kieselsäuregels

gesprüht wurde, innig vermischt. Man erhält auf diese Weise eine Aufbereitung des Wirkstoffs mit guter Haftfähigkeit.

VIII. 20 Teile des Wirkstoffs Nr. 153 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der sie enthaltenden Mittel kann im Vorauflaufverfahren oder im Nachauflaufverfahren erfolgen. Sind die Wirkstofie für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadien, Bodenart und Anwendungsverfahren 0,025 bis 3 kg/ha, vorzugsweise 0,1 bis 1 kg/ha.

Die herbizide Wirkung der Cyclohexan-1,3-dionderivate der Formel I läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Bei dieser Applikationsmethode beträgt die Aufwandmenge 3,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Die Sojapflanzen werden in einem mit Torfmull angereicherten Substrat angezogen. Es werden entweder direkt gesäte und in gleichen Gefäßen aufgewachsene Pflanzen verwendet, oder aber solche, die erst als Keimpflanzen getrennt angezogen und einige Tage vor der Behandlung in die Versuchsgefäße verpflanzt werden. Die Aufwandmenge beträgt 0,25 und 0,5 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:.

Alopecurus myosuroides (Ackerfuchsschwanz), Avena fatua (Flughafer), Bromus inermis (unbegrannte Trespe), Echinochloa crus-galli (Hühnerhirse), Glycine max (Sojabohnen), Hordeum vulgare (Gerste), Lolium multiflorum (Ital. Raygras), Setaria italica (Kolbenhirse), Sorghum halepense (Sudangras), Triticum aestivum (Weizen).

Bei der Vorauflaufanwendung im Gewächshaus zeigen beispielsweise die Verbindungen Nr. 2, 4, 9, 14, 17, 18, 90, 94, 113, 116, 117, 125 und 153 bei einer Aufwandmenge von 3,0 kg Wirkstoff/ha eine beachtliche herbizide Aktivität gegen Gräser.

Bei Nachauflaufbehandlung sind beispielsweise die Verbindungen Nr. 4, 6, 17, 38, 90, 94, 113, 116 und 117 mit 0,25 kg Wirkstoff/ha in breitblättrigen Kulturen selektiv herbizid wirksam. Die Verbindungen Nr. 2, 9 und 10 sowie 153 und 155 bekämpfen beispielsweise unerwünschte Gräser in Getreiden bei Aufwandmengen von 0,5 kg bzw. 0,25 kg Wirkstoff/ha.

In Anbetracht der Verträglichkeit der Applikationsmethoden können die erfindungsgemäßen Verbindungen noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var, silvestris | Rübsen |
| Camellia sinensis | Teestrauch |
| Carthamus tinctorius | Saflor - Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffes liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glyoine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |

| | |
|---|---|
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Rartoffel |
| Sorghum bicolor (s. vulgare) | Mohrenhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |

Zur Verbreiterung des Wirkungsspektrums und zur Erzielung synergistischer Effekte können die Cyclohexan-1,3-dionderivate der Formel I bzw. die sie enthaltenden Mittel auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Triazinone, Uracile, Benzofuranderivate, andere Cyclohexan-1,3-dionderivate und andere in Betracht.

Außerdem kann es von Nutzen sein, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nichtphytotoxische Öle und Ölkonzentrate zugesetzt werden.

## Patentansprüche

1. Cyclohexan-1,3-diondervate in allen tautomeren Formen der Formel

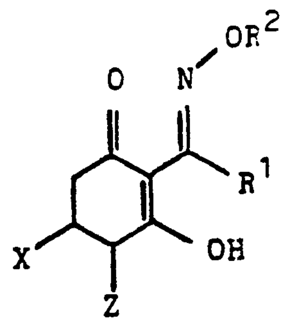

(I),

in der

$R^1$ $C_1$-$C_4$-Alkyl,

$R^2$ $C_1$-$C_4$-Alkyl, gegebenenfalls halogensubstituiertes $C_3$-$C_5$-Alkenyl oder $C_3$-$C_5$-Alkinyl,

X einen gegebenenfalls ungesättigten fünf- oder sechsgliedrigen Heterocyclus, der mindestens einen $C_1$-$C_4$-Alkoxy-, $C_1$-$C_4$-Alkyl-thio-, $C_5$-$C_7$-Cycloalkoxy- oder Benzoxyrest oder einen gegebenenfalls durch Halogen, $C_1$-$C_4$-Alkyl oder $C_1$-$C_4$-Alkoxy substituierten Phenyl-, Phenoxy- oder Phenylthiorest als Substituent tragt und außerdem durch Alkylreste substituiert sein kann, und

Z Wasserstoff, Methoxycarbonyl, Ethoxycarbonyl, Methyl oder Cyano bedeuten,

sowie Salze dieser Verbindungen.

2. Cyclohexan-1,3-dionderivate der Formel I gemäß Anspruch 1, dadurch gekennzeichnet, daß X einen gegebenenfalls ungesättigten fünf- oder sechsgliedrigen Heterocyclus, der mindestens einen $C_1$-$C_4$-Alkoxyrest als Substituenten trägt, bedeutet.

3. Cyclohexan-1,3-dionderivate der Formel I gemäß Anspruch 1, dadurch gekennzeichnet, daß Z Wasserstoff bedeutet.

4. Cyclohexan-1,3-dionderivate der Formel I, gemäß Anspruch 1, dadurch gekennzeichnet, daß der Heterocyclus im Substituenten X ein Tetrahydropyranylrest ist.

5. Verfahren zur Herstellung eines Cyclohexan-1,3-dionderivates der Formel I gemäß Anspruch 1, dadurch gekennzeichnet, daß man eine Verbindung der Formel

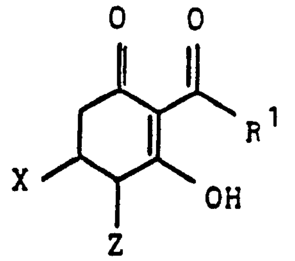

(II),

in der $R^1$, Z und X die in Anspruch 1 genannten Bedeutungen haben,

a) mit einer Ammoniumverbindung der Formel $R^2ONH_3Y$, in der $R^2$ die in Anspruch 1 genannten Bedeutungen hat und Y ein Anion bedeutet, in einem inerten Lösungsmittel bei einer Temperatur zwischen 0 und 80°C gegebenenfalls in Gegenwart einer Base umsetzt oder

b) mit einem gegebenenfalls in wäßriger Lösung vorliegenden Hydroxylamin der Formel $R^2ONH_2$, wobei $R^2$ die in Anspruch 1 genannten Bedeutungen hat, bei einer Temperatur zwischen 0 und 80° in Gegenwart eines Lösungsmittels umsetzt oder

c) mit unsubstituiertem Hydroxylammoniumsalz $NH_2OH$ . HY, wobei Y ein beliebiger Anion bedeutet, bei einer Temperatur zwischen 0 und 80°C in Gegenwart eines Lösungsmittels und einer Base umsetzt und das erhaltene Oxim der Formel

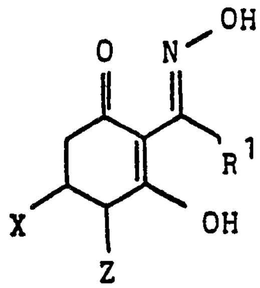

(III)

in der $R^1$, X und Z die in Anspruch 1 genannten Bedeutungen haben, mit einem Alkylierungsmittel der Formel

$R^2$-Y' (IV),

in der Y' eine Abgangsgruppe bedeutet und $R^2$ die in Anspruch 1 genannten Bedeutungen hat, alkyliert.

6. Herbizid, enthaltend ein Cyclohexan-1,3-dionderivat der Formel I gemäß Anspruch 1.

7. Herbizid, enthaltend inerte Zusatzstoffe und ein Cyclohexan-1,3-dionderivat in allen Tautomeren Formen der Formel

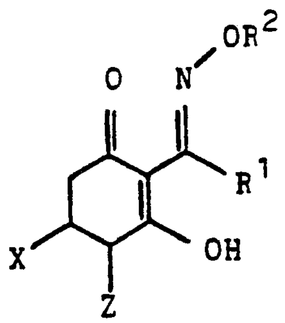

in der

$R^1$ $C_1$-$C_4$-Alkyl,

$R^2$ $C_1$-$C_4$-Alkyl, gegebenenfalls halogensubstituiertes $C_3$-$C_5$-Alkenyl oder $C_3$-$C_5$-Alkinyl,

X einen gegebenenfalls ungesättigten fünf- oder sechsgliedrigen Heterocyclus, der mindestens einen $C_1$-$C_4$-Alkoxy-, $C_1$-$C_4$-Alkylthio-, $C_5$-$C_7$-Cycloalkoxy- oder Benzoxyrest oder einen gegebenenfalls durch Halogen, $C_1$-$C_4$-Alkyl oder $C_1$-$C_4$-Alkoxy substituierten Phenyl- Phenoxy- oder Phenylthiorest als Substituent trägt und außerdem durch Alkylreste substituiert sein kann, und

Z Wasserstoff, Methoxycarbonyl, Ethoxycarbonyl, Methyl oder Cyano bedeuten, oder Salze dieser Verbindungen als Wirkstoff.

8. Herbizid nach Anspruch 6, dadurch gekennzeichnet, daß es ein Cyclohexan-1,3-dionderivat der Formel I enthält, wobei X einen gegebenenfalls ungesättigten fünf - oder sechsgliedrigen Heterocyclus, der mindestens einen $C_1$-$C_4$-Alkoxyrest als Substituenten trägt, bedeutet.

9. Herbizid nach Anspruch 6, dadurch gekennzeichnet, daß es ein Cyclohexan-1,3-dionderivat der Formel I enthält, wobei Z Wasserstoff bedeutet.

10. Verfahren zur Bekämpfung unerwünschten Pflanzenwuchses, dadurch gekennzeichnet, daß man die unerwünschten Pflanzen oder die von unerwünschtem Pflanzenwachstum freizuhaltende Fläche mit einer herbizid wirksamen Menge eines Cyclohexan-1,3-dionderivates der Formel I gemäß Anspruch 1 behandelt.

## Claims

1. A cyclohexane-1,3-dione derivative, in any tautomeric form, of the formula

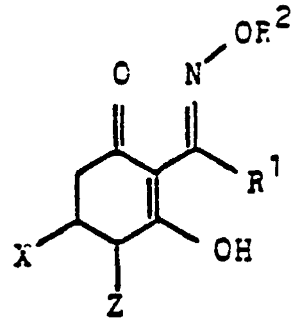

(I),

where $R^1$ is $C_1$-$C_4$-alkyl, $R^2$ is $C_1$-$C_4$-alkyl, unsubstituted or halogen-substituted $C_3$-$C_5$-alkenyl or $C_3$-$C_5$-alkynyl, X is a saturated or unsaturated five-membered or six-membered heterocyclic structure which is substituted by one or more $C_1$-$C_4$-alkoxy, $C_1$-$C_4$-alkylthio, $C_5$-$C_7$-cycloalkoxy or benzyloxy radicals, or by a phenyl, phenoxy or phenylthio radical which is unsubstituted or substituted by halogen, $C_1$-$C_4$-alkyl or $C_1$-$C_4$-alkoxy, and may furthermore be substituted by alkyl, and Z is hydrogen, methoxycarbonyl, ethoxycarbonyl, methyl or cyano, and salts of this compound.

2. A cyclohexane-1,3-dione derivative of the formula I as claimed in claim 1, wherein X is a saturated or unsaturated five-membered or six-membered heterocyclic structure which is substituted by one or more $C_1$-$C_4$-alkoxy radicals.

3. A cyclohexane-1,3-dione derivative of the formula I as claimed in claim 1, wherein Z is hydrogen.

4. A cyclohexane-1,3-dione derivative of the formula I as claimed in claim 1, wherein the heterocyclic structure X is tetrahydropyranyl.

5. A process for the preparation of a cyclohexane-1,3-dione derivative of the formula I as claimed in claim 1, wherein a compound of the formula

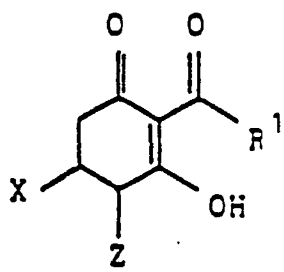

(II),

where $R^1$, Z and X have the meanings given in claim 1 is reacted

a) with an ammonium compound of the formula $R^2ONH_3Y$, where $R^2$ has the meanings given in claim 1 and Y is an anion, in an inert solvent, at from 0 to 80°C, and in the presence or absence of a base; or

b) with a hydroxylamine, if desired, in aqueous solution, of the formula $R^2ONH_2$, where $R^2$ has the meanings given in claim 1, at from 0 to 80°C and in the presence of a solvent; or

c) with an unsubstituted hydroxylammonium salt $NH_2OH.HY$, where Y is any anion, at from 0 to 80°C and in the presence of a solvent and a base,

and the oxime of the formula

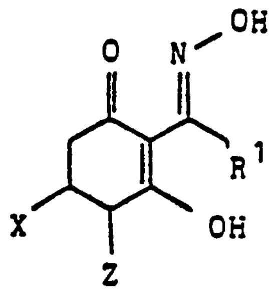

(III)

where $R^2$, X and Z have the meanings given in claim 1, which is obtained is alkylated with an alkylating agent of the formula

$R^2$-Y' (IV),

where Y' is a leaving group and $R^2$ has the meanings given in claim 1.

6. A herbicide containing a cyclohexane-1,3-dione derivative of the formula I as claimed in claim 1.

7. A herbicide containing inert additives and a cyclohexane-1,3-dione derivative, in any tautomeric form, of the formula

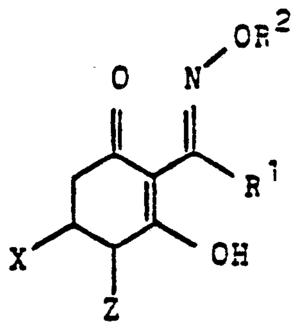

where $R^1$ is $C_1$-$C_4$-alkyl, $R_2$ is $C_1$-$C_4$-alkyl, unsubstituted or halogen-substituted $C_3$-$C_5$-alkenyl or $C_3$-$C_5$-alkynyl, X is a saturated or unsaturated five-membered or six-membered heterocyclic structure which is substituted by one or more $C_1$-$C_4$-alkoxy, $C_1$-$C_4$-alkylthio, $C_5$-$C_7$-cycloalkoxy or benzyloxy radicals, or by a phenyl, phenoxy or phenylthio radical which is unsubstituted or substituted by halogen, $C_1$-$C_4$-alkyl or $C_1$-$C_4$-alkoxy, and may furthermore be substituted by alkyl, and Z is hydrogen, methoxycarbonyl, ethoxycarbonyl, methyl or cyano, or a salt of this compound, as an active ingredient.

8. A herbicide as claimed in claim 6, which contains a cyclohexane-1,3-dione derivative of the formula I, where X is a saturated or unsaturated five-membered or six-membered heterocyclic structure which is substituted by one or more $C_1$-$C_4$-alkoxy radicals.

9. A herbicide as claimed in claim 6, which contains a cyclohexane-1,3-dione derivative of the formula I, where Z is hydrogen.

10. A process for controlling undesirable plant growth, wherein the undesirable plants or the area to be kept free from undesirable plant growth are treated with a herbicidally effective amount of a cyclohexane-1,3-dione derivative of the formula I as claimed in claim 1.

**Revendications**

1. Dérivés de la cyclohexane-1,3-dione, dans toutes les formes tautomères, de la formule

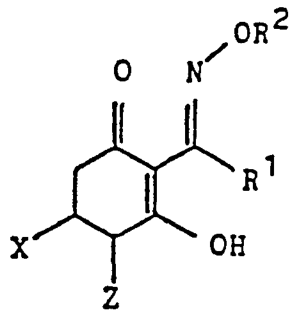

(I),

dans laquelle

$R^1$ désigne un radical alkyle en $C_1$ à $C_4$,

$R^2$ désigne un radical alkyle en $C_1$ à $C_4$ ou un radical alcényle en $C_3$ à $C_5$ ou alcynyle en $C_3$ à $C_5$ éventuellement halo-substitué,

X représente un composé hétérocyclique penta- ou hexagonal éventuellement non saturé, qui porte au moins un substituant choisi parmi les groupes alcoxy en $C_1$ à $C_4$, alkyl-thio en $C_1$ à $C_4$, cycloalcoxy en $C_5$ à $C_7$ et benzoxy, ainsi que les groupes phényle, phénoxy et phénylthio éventuellement halo-, alkyl(en $C_1$ à $C_4$)-ou alcoxy(en $C_1$ à $C_4$)-substitués, et qui peut en outre être substitué par des radicaux alkyle et

Z représente un atome d'hydrogène ou un groupe méthoxycarbonyle, éthoxycarbonyle, méthyle ou cyano, ainsi que les sels de ces dérivés.

2. Dérivés de la cyclohexane-1,3-dione de la formule I suivant la revendication 1, caractérisés en ce que X représente un composé hétérocyclique penta- ou hexagonal, saturé ou non saturé, qui porte comme substituant au moins un radical alcoxy en $C_1$ à $C_4$.

3. Dérivés de la cyclohexane-1,3-dione de la formule I suivant la revendication 1, caractérisés en ce que Z représente un atome d'hydrogène.

4. Dérivés de la cyclohexane-1,3-dione de la formule I suivant la revendication 1, caractérisés en ce que le substituant hétérocyclique X est un groupe tétrahydropyrannyle.

5. Procédé de préparation d'un dérivé de la cyclo-hexane-1,3-dione de la formule I suivant la revendication 1, caractérisé en ce que l'on fait réagir un composé de la formule

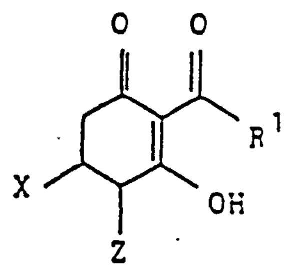

(II),

dans laquelle $R^1$, Z et X possèdent les significations définies dans la revendication 1,

a) dans un solvant inerte et éventuellement en présence d'une base, à une température comprise entre 0 et 80°C, avec un dérivé d'ammonium de la formule $R^2ONH_3Y$, dans laquelle $R^2$ possède les significations définies dans la revendication 1 et Y représente un anion , ou

b) dans un solvant, à une température entre 0 et 80°C, avec une hydroxyl-amine de la formule $R^2ONH_2$, dans laquelle $R^2$ possède les significations définies dans la revendication 1, éventuellement en solution aqueuse, ou

c) dans un solvant et en présence d'une base, à une température comprise entre 0 et 80°C, avec un sel de l'hydroxyl-ammonium non substitué de la formule $NH_2OH.HY$, dans laquelle Y désigne un anion quelconque,

puis on soumet l'oxime obtenue, de la formule

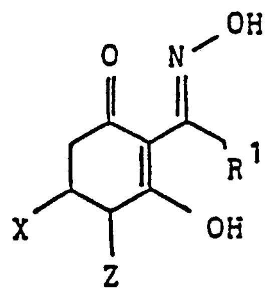

(III),

dans laquelle $R^1$, X et Z possèdent les significations définies dans la revendication 1, à une alkylation par un agent alkylant de la formule

$R^2$ - Y' (V)

dans laquelle Y' représente un groupe à cliver et $R^2$ possède les significations définies dans la revendication 1.

6. Composition herbicide, contenant un dérivé de la cyclohexane-1,3-dione de la formule I suivant la revendication 1.

7. Composition herbicide, contenant un dérivé de la cyclohexane-1,3-dione, dans toutes ses formes tautomères, de la formule

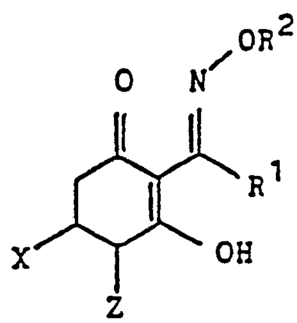

,

dans laquelle

$R^1$ désigne un radical alkyle en $C_1$ à $C_4$,

$R^2$ désigne un radical alkyle en $C_1$ à $C_4$ ou un radical alcényle en $C_3$ à $C_5$ ou alcynyle en $C_3$ à $C_5$ éventuellement halo-substitué,

X représente un composé hétérocyclique penta- ou hexagonal éventuellement non saturé, qui porte au moins un substituant choisi parmi les groupes alcoxy en $C_1$ à $C_4$, alkyl-thio en $C_1$ à $C_4$, cycloalcoxy en $C_5$ à $C_7$ et benzoxy, ainsi que les groupes phényle, phénoxy et phénylthio éventuellement halo-, alkyl(en $C_1$ à $C_4$)-ou alcoxy(en $C_1$ à $C_4$)-substitués, et qui peut en outre être substitué par des radicaux alkyle et

Z représente un atome d'hydrogène ou un groupe méthoxycarbonyle, éthoxycarbonyle, méthyle ou cyano, ou des sels de ce dérivé comme principe actif.

8. Composition herbicide suivant la revendication 6, caractérisée en ce qu'elle contient un dérivé de la cyclohexane-1,3-dione de la formule I , pour lequel X est un composé hétérocyclique penta- ou hexagonal, saturé ou non saturé, qui porte au moins un radical alcoxy en $C_1$ à $C_4$ comme substituant.

3. Composition herbicide suivant la revendication 6, caractérisée en ce qu'elle contient un dérivé de la cyclohexane-1,3-dione de la formule I, pour lequel Z = H.

10. Procédé pour combattre le développement de plantes indésirables, caractérisé en ce que l'on traite les plantes indésirables ou la surface, sur laquelle la croissance de ces plantes indésirables doit être inhibée, avec une quantité efficace du point de vue herbicide d'un dérivé de la cyclohexane-1,3-dione de la formule I suivant la revendication 1.